# EUROPEAN PATENT SPECIFICATION

(11) **EP 1 701 652 B1**
(45) Date of publication and mention of the grant of the patent: **17.04.2013**
(21) Application number: 04770628.8
(22) Date of filing: 24.10.2004
(51) Int. Cl.: A61B 3/16

(54) **DEVICE FOR MEASUREMENT OF INTRA OCCULAR PRESSURE**
VORRICHTUNG ZUR MESSUNG DES AUGENINNENDRUCKS
DISPOSITIF DE MESURE DE LA PRESSION INTRA-OCULAIRE

(30) Priority: 24.10.2003 US 513696 P
(43) Date of publication of application: 20.09.2006
(73) Proprietor: Tonoguard Ltd., 38100 M.P. Hefer (IL)
(72) Inventor: GUR, Joshua, 97752 Jerusalem (IL); HAIM-CHOUMLA, Ezra, 60920 Kadima (IL); BARASH, David, 64682 Tel Aviv (IL)
(74) Representative: Messulam, Alec Moses
(86) International application number: PCT/IL2004/000965
(87) International publication number: WO 2005/039379

(56) References cited:
- EP-A- 0 267 022
- DE-A1- 4 213 360
- JP-A- 2003 299 622
- US-A- 3 595 849
- US-A- 5 727 551
- US-A- 6 110 110
- US-A1- 2002 173 712

## Description

### TECHNICAL FIELD OF THE INVENTION

The present invention relates generally to tonometry and more particularly to non-contact tonometry. The invention also relates to measurement of intra ocular pressure (IOP) and to devices for measuring same.

### BACKGROUND OF THE INVENTION

The significance of measurement of the hydrostatic intra-ocular pressure (IOP) is well known in medicine (ophthalmology). Excessive Internal eye pressure is a cause of glaucoma and other eye diseases.

Professional medical staff, using a variety of methods, mainly indentation tonometry, applanation tonometry or non-contact tonometry, perform common measurements of IOP. Available in the market are several systems. An example of an indentation tonometry system is the Schiotz tonometer, which dates back to the 1930's. A more recent system is the Mentor Tono-Pen XL from Mentor O&O, Inc. of Norwell, MA, USA. An example of an applanation tonometry system is the Goldman tonometer, which is a standard tonometer in a considerable amount of medical institutions. The above mentioned systems and methods are based on direct contact with the corneal eye associated with liquids and medications applied to the eye. A non-contact tonometry is preferable since it decreases hazards to the tested eye associated with the measurement process. An example of a non-contact tonometry system is the puff tonometer XPERT NCT from Reichert, Cambridge, MA, USA. Puff tonometry employs detecting changes in the reflectance of a cornea, which is distorted by a pneumatic pulse. The measurement process basically includes the steps of: (i) placing and aligning the tonometer in front of the tested eye; (ii) measuring the intensity of a light reflected by a test area within the surface of the cornea; (iii) distorting the cornea by projecting a pneumatic pulse towards it, and (iv) matching features of the intensity-time profile of the light reflected by the distorted cornea with IOP values. The accuracy of IOP values obtained is considerably affected by misplacement or misalignment of the tonometer. Therefore allowed tolerances of placement and alignment are significantly narrow.

Once a patient is diagnosed as having an excessive eye pressure, he/she should have his/her IOP monitored periodically ù typically at different hours during the day. It is desirable that the patient is able to measure his or her own IOP at home, rather than make a special visit to a clinic for this purpose. However, current commercially available devices providing for self-examination of IOPI5are not satisfactory mainly due to accuracy issues and cost considerations.

Therefore, efforts to develop a self-operated tonometer suitable for home use are on-going. US patent 6,440,070 discloses a device consisting of a gauge, which is pushed against the eyelid while force is measured. An ultrasound-measuring device is also used to measure the distance from an internal object within the tested eye. Correlating the force applied to the displacement of the pressed surface caused by this applied force provides assessment of pressure. US patent 6,746,400 discloses a system implementing a plurality of pressure sensors which also provides a spatial distribution of pressure from which IOP can be derived. Both above mentioned patents involve direct or indirect contact with the tested eye. Measured IOP values obtained in such manner are considerably less sensitive to fitting or aligning of the measurement equipment as compared to those obtained by systems employing optical measurements. However, patients tend to decline using a measuring system which entails applying force by touching the eye.

### SUMMARY OF THE INVENTION

According to a first aspect of the invention, there is provided a device for measuring internal pressure of a body, comprising a light collecting and delivering device formed of a wall surrounding a lumen, the wall having an end surface for receiving light reflected by the body, the light being guided to travel along the wall by total internal reflection within the wall, an illuminating beam source for providing a light beam to the body, the light beam passing through the lumen of the light collecting and delivering device; an air source for providing a pneumatic pulse of compressed air to the body by way of the lumen of the light collecting and delivering device to flatten a convex surface of the body; a light detector mounted to receive light reflected by the body and passing through the wall of the light collecting and delivering device, a reflector provided between the lumen and the light detector to reflect the illuminating light beam into the lumen and to prevent light reflected by the body into the lumen from reaching the detector, and a control unit connected to the detector to provide a measure of the internal pressure of the body.

According to a second aspect of the invention, there is provided a method of measuring internal pressure of a body having a convex surface which comprises aligning with the body a longitudinal axis of a light collecting and delivering device formed of a wall surrounding an axially extending lumen; illuminating the body by way of the light collecting and delivering device and applying thereto a pneumatic pulse capable of substantially flattening the body, the illuminating light beam and the pneumatic pulse passing axially through the lumen, guiding by total internal reflection through the wall of the light collecting and delivering device light reflected from the body to a detector when the body is in a non-flattened configuration, the light when the body is in a flattened configuration being reflected into the lumen and prevented from reaching the detector; and matching a time-related feature associated with changes in the light intensity of reflected light delivered to the detector, with a given pressure value related to the pneumatic pulse, the time related feature being at least one of a time interval in which the changes of the light intensity are detected and a slope of the changes of the light intensity.

In contrast with other prior art proposals discussed below, in both aspects of the invention, the object is illuminated by means of a beam that passes along the same lumen as the pneumatic pulse. A tubular light guiding device surrounding the lumen is used to transmit the light reflected by the body to the detector while light reflected back into the lumen when the body is flattened does not reach the detector.

US 5,727,551 delivers a pneumatic pulse along the optical axis of the tonometer but does not use a tubular light guiding device to transmit the reflected light to a detector. Instead a complex system of optics is employed which would preclude operation by the patient. Furthermore, light reflected when the body has been flattened into the hole through which the pneumatic pulse is applied will reach the detector.

DE 4213360, believed to represent the closest prior art to the present invention, uses a tubular light guide surrounding a lumen through which a pneumatic pulse is applied. However, in this proposal, the illuminating beam does not pass through the lumen. Instead, one side of the tubular light guide is used to transmit an illuminating beam and the other side is used to transmit reflected light to the detector.

EP 0267022 shows a tonometer that has a hand held unit in which a pneumatic pulse is applied through a hole in a lens that is used both to illuminate the eye and to sense the reflected light. The unit however relies on conventional optics with lenses and mirrors to transmit reflected light to the detectors and does not prevent light reflected when the eye is flattened from reaching the detector.

### BRIEF DESCRIPTION OF THE DRAWINGS

Fig. 1 is a schematic side view of a non-contact tonometer as applied to patient according to a preferred embodiment of the present invention;
Fig. 2 is a schematic longitudinal sectional view of a segment of an optimal unit of the non-contact tonometer of Fig. 1;
Fig. 3 is a graph depicting simulated pressure of compressed air and intensity of reflected light versus time for the present tonometer; and
Fig. 4 is a block diagram of a control unit usable with the tonometer of the present invention.

### DETAILED DESCRIPTION OF THE PRESENT INVENTION

The present invention provides a method and a device for determining internal pressure within deformable bodies by measuring the manner in which light is reflected by the body in response to mechanical disturbance applied to the body. The method provides considerably wider tolerances in placement and alignment of the tonometer related to the test area without sacrificing the accuracy of essential measurements. The method is suitable for measuring internal pressure within most kinds of deformable bodies having a convex surface and in particular for measuring intra ocular pressure (IOP).

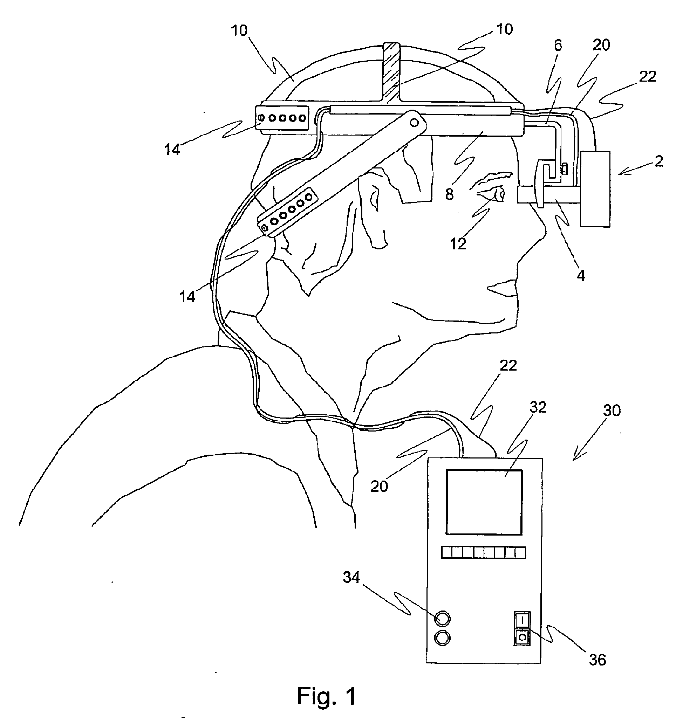

Reference is made to Fig. 1 showing a tonometer adapted for measurement of IOP, according to a preferred embodiment of the present invention. Electro-optical unit 2 having a cylindrical extension 4 is placed in front of a patient. The electro-optical unit 2 is attached to a mounting device by means of a connecting device 6 consisting of frame 8, which is placed over the patient's head. Straps 10 support frame 8 over the head so that the aperture of electro-optical unit 2 faces the eye 12. The unit 2 is fixed in place by means of adjustable straps 14 at the patient's forehead. The mounting device of this tonometer consists of frame 8, straps 10, straps 14 and connecting device 6. The connecting device 6 is slidably attached to frame 8 in front of either the left or right eye by means of a clamping device or other fastener (not shown). A flexible tube 20 conveys compressed air; and a power and signal cable 22 connects electro-optical unit 2 with a control unit 30 of the tonometer. A display 32 on which operating instructions and measurement results are shown is located at the front panel of the control unit 30. Functional keys 34 and a main operating switch 36 are also installed on this front panel.

After the user (patient) attaches the electro-optical unit 2 to frame 8 in its pre- assigned place for attending to the left or right eye, operating switch 36 is actuated and the user mounts frame 8 over his/her head and adjusts its placement by means of the adjustable straps 14 to fit in front of the selected eye. Upon activating the system, an illuminating beam is emitted from an optical aperture located on the side of the electro-optic unit 2 facing the user. By pressing a functional key 34 the user hears a sound changing its pitch and/or watches the display 32, on which the intensity of reflected light from the surface of his eye is displayed, in order to maximize the intensity and improve placement and pointing of the electro-optical unit 2 in front of his eye. The patient can also activate a reticule by pressing another functional key 34 and center it in his field of view, by which the test area is centered over the corneal apex. The frame 8 is adjusted to the user forehead by means of adjustable supporting straps 14 for securing the position of the unit 2 and keeping it stable on the user.

Different mounting configurations are possible, as long as the electro-optical unit 2 remains stably mounted on the patient. Another preferred embodiment of a self- operated tonometer according to the present invention takes the form of goggles (not shown) attached to the user's head by means of a flexible strap. In such a case, the electro-optical unit 2 is attached to the goggles corresponding to the selected eye by means of clamping connectors.

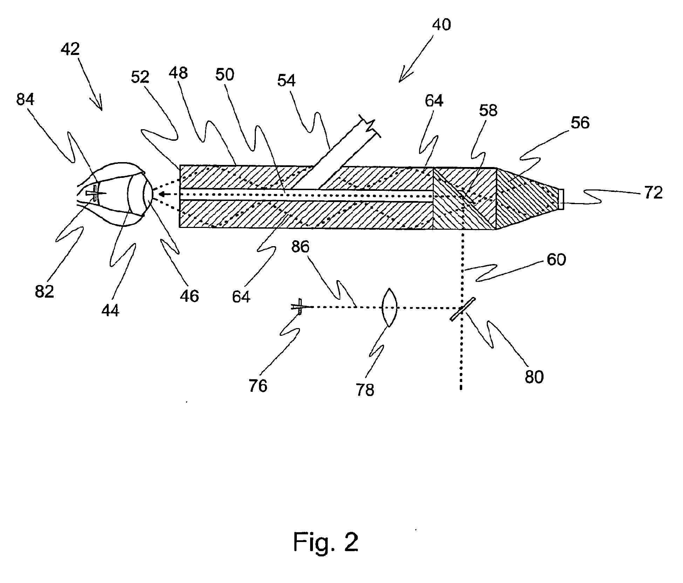

Reference is now made to Fig. 2, showing a schematic longitudinal sectional view of a segment 40 of the electro-optical unit 2 of a tonometer of the invention. Segment 40 of the electro-optical unit 2 is placed in front of a user's eye 42. IOP is typically measured in the area of the cornea 44, preferably at its center, (which is the location of the pupil). Therefore the electro-optical unit 2 is pointed toward the cornea 44 on which a substantially central sector 46 is to be illuminated. In a preferred embodiment of the invention, there is provided a unitary device which is both a light projecting element and a collecting tube (LPCT). The LPCT 48 is positioned in front of the pupil taking care not to touch the eyelashes. LPCT 48 has a substantially thick wall defining a narrow lumen 50. The proximal face 52 of LPCT 48 is coated with a suitable anti-reflecting coating. The flexible tube 54 is connected to the lumen 50 by way of an aperture in the wall of LPCT 48. The flexible tube 54 is connected, at its other end, to a source of compressed air (not shown). A light coupling device 56 is attached to the distal face of LPCT 48 by means of a refraction index- matching glue or any suitable glue such as EPOTEC« (a commonly used optical glue). The proximal face 52 of the light coupling device 56 conforms with the distal face of LPCT 48. The light coupling device 56 assumes the shape of a frustum of a pyramid or a cone towards its distal end. A light reflector 58 consisting of a highly reflective, diagonally mounted plate, is attached to the inner surface of the light-coupling device 56 covering the lumen 50 completely. This reflector 58 deflects an illuminating beam 60 directing it along the axis of LPCT 48 to the central sector 46. Different variants of the reflector 58 and its mount are applicable, preferably substantially reflecting the entire illuminating beam 60 towards the eye 42. The light source emits a collimated light, typically from a light emitting diode (LED), not shown. The wall of LPCT 48 is transparent in the spectral range of the illuminating beam 60 and has a suitable index of refraction to act as a light guide. Light beams 64 reflected from the central sector 46 of the eye 42, are guided by total internal reflection along the walls of LPCT 48 and further propagate through the light coupling device 56, impinging on a light detector 72. Reflector 58 blocks light reflected from the eye 42 from reaching light detector 72 directly through the lumen 50. Light detector 72 is attached to the distal face of light coupling device 56 normal to the axis of LPCT 48.

The reflected light receiving surfaces include the coated proximal face 52 of the wall of LPCT 48, reflector 58 and light detector 72. The positioning of light reflector 58 considerably reduces the contribution of light reflected by the corneal apex to the total intensity received by the light detector 72. The electro-optical system 2 of the invention is less sensitive to the alignment with the face of the user. The fact that the illuminating beam 60, pneumatic pulse and the detector 72 share the same axis makes the system less vulnerable to axis misalignments.

The wall of LPCT 48 acts to guide the light 64 reflected by the eye to the detector 72 by total internal reflection within the wall .

Mounting the tonometer to the user's head is followed by aligning it on the user's face, regulated by maximizing the intensity of reflected light 64 received by the detector 72. Such a procedure is more suitable for a skilled and trained operator. In order to make the tonometer of the present invention suitable for self-examination of IOP, a reticule 76 is used to simplify the alignment and attaching steps. The patient observes reticule 76 through a lens 78 and a beam splitting device 80. When the user has a reticule image 82 centered in the field of view on his/her retina 84 the operating switch 36 is actuated, triggering the measurement process. The intensity of light reflected from the cornea 44 is measured by the detector 72, connected to an electronic subsystem (not shown). The control unit 30 of the system subsequently sends an instruction to an air switch (neither shown) to release a pulse of compressed air through the flexible tube 54 and subsequently to the lumen 50. The pulse of compressed air, which is directed at central sector 46, transiently deforms the cornea 44 distorting its curvature, making it temporarily flatter or even concave. The illuminating beam 60 illuminates the eye 42 where the cornea 44 has been distorted and the changes in geometry yield correlated changes in the amount of reflected light 64 that is captured by proximal face 52 of LPCT 48. This reflected light is then detected by light detector 72, which translates the changes in light intensity into changes in an electric signal. This electric signal is further sampled and digitized yielding a series of digital values which are sent to the control unit 30 for analysis.

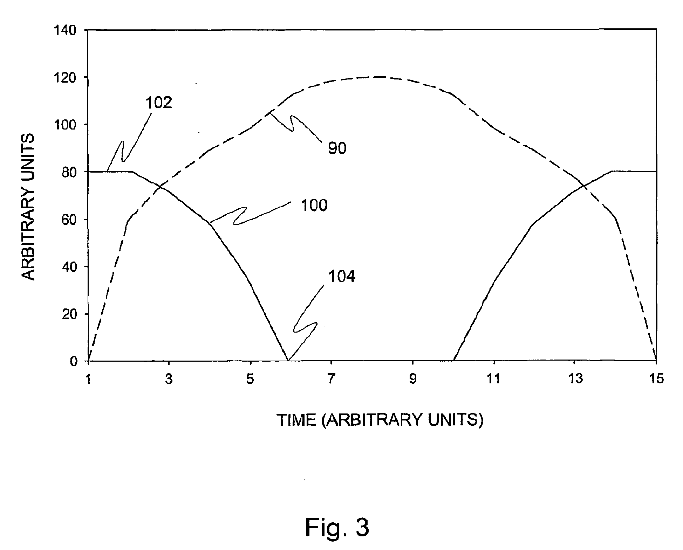

Reference is now made to Fig. 3 showing a graph 90 of simulated pressure readings and intensity of reflected light versus time as generated by the non-contact tonometer of the invention. Pressure values 90 represent measurements made inside the flexible tube 54 near the aperture. The intensity of reflected light at the face of light detector 72 is represented by plot 100. At first, the intensity of received reflected light is maximal, as indicated by segment 102, which corresponds to reflection from the undistorted cornea 44. The larger volume of the lumen of tube 50 and the diversion of the compressed air as it passes the aperture cause a pressure rise at the cornea surface to lag behind in time with respect to the pressure rise at the measuring point. Therefore the cornea 44 maintains its undistorted curvature as the pressure values rise at the measuring point. Flattening of the cornea starts a while after the pneumatic pulse is initiated and similarly the cornea 44 resumes its original curvature somewhat before the pressure change is nullified. The cornea 44 is flattened at point 104 of plot 100 in which the intensity is zeroed. The cornea 44 is illuminated with a narrow illuminating beam 60 emitted from lumen 50. A convex surface of the undistorted cornea 44 implies a larger effective area, which reflects light back towards face 52. A planar surface has smaller effective area, which reflects this illuminating beam 60 toward face 52 of LPCT 48, and therefore correlates with a lower light intensity reflected. When a certain threshold of low reflection intensity is reached, a zero reflectance value is assigned and any lower light intensity reflected is interpreted as zero, even as the cornea 44 assumes a concave configuration.

Features of the intensity-time profile indicated in plot 100 are correlated with actual IOP values. IOP values are correlated with the time intervals in which light intensity changes from its maximal value to zero and back again to its maximal value. Higher IOP values correspond to longer time intervals, at the falling light intensity and shorter time interval at the rising edge of light intensity, at a given pressure-time profile. Therefore, measuring this time interval and comparing it with equivalent time intervals measured related to eyes with a given IOP, results in an IOP value. The IOP value of the measured body is determined by associating such time-related features of the measured curve with a given IOP value. These time-related features associated with IOP values are pre-stored in the control unit 30. Therefore, IOP is determined by matching time-related features of measured changes in light intensity with pre-stored IOP values.

An IOP value is displayed followed by a message indicating to the user that the measurement process has proceeded properly. Fault indications are displayed when signal intensity, or pressure values exceeds its typical ranges. They user is instructed to activate a built in self-examination procedure and repeat the measurement process if the display 32 indicates that the tonometer has not functioned properly. The process is repeated by checking the mounting of the tonometer in front of the eye 42 and centering the reticule image 82 in the field of view.

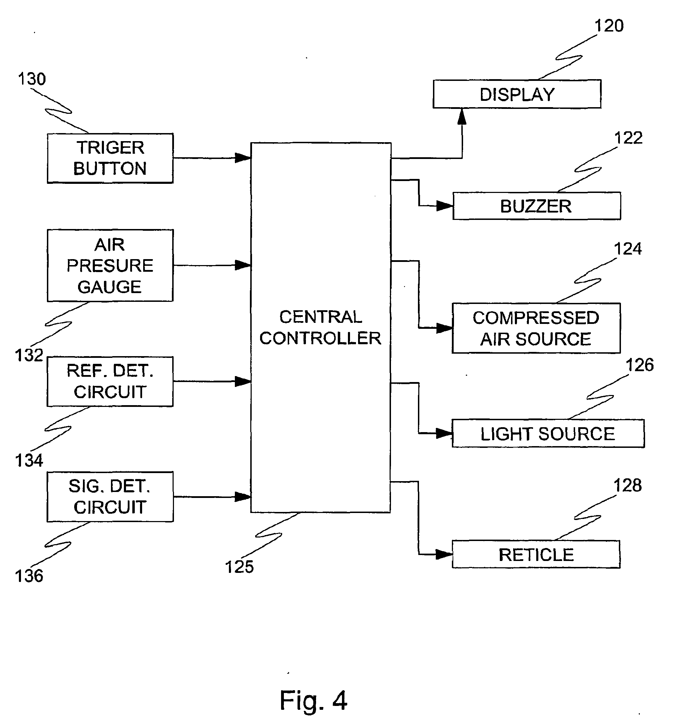

Reference is now made to Fig. 4 showing a block diagram of the electronics sub-system of a preferred embodiment of the present invention. A display 120 displays instructions to the user as well as results of the measurements. A suitable display device is, for example, an LED/LCD display and or voiced instructions. The sub-system may include a buzzer 122 which makes an audible sound when the attention of the user has to be called. This may happen if a measurement fails, or if the battery is low, etc. The buzzer 122 changes its pitch following the intensity of the received signal when aligning the LPCT 48 with the cornea 44. A compressed air valve 124 operates on a command from the central controller 125, 30, to project a pneumatic pulse into the system. The driver of illuminating light source 126 and reticule 128, 76 are controlled by the central controller 125. A trigger button 130, 36, why is also connected to the central controller 125, initiates the measurement process when pressed by the user after placing and aligning the optical unit 2 with the eye 12, 42 to be tested. A safety air pressure gauge 132 is used in the compressed air line, to stop the airflow if it exceeds a specified flow or duration. A light detector circuitry of a reference detector circuit 134 and of the signal detector 136, include synchronized sampling and digitizing means implemented by suitable sample and hold devices and analog to digital converters, and are connected to the central controller feeding it with data. The reference detector circuit 134 processes the information from a reference detector. The signal detector circuit 136 picks up the information from the light detector 72, triggered by the reference signal created at the reference detector circuit 134. This optional reference trigger arrangement enables this detector circuit 134 to detect very weak signals and thus be very sensitive.

The tonometer according to this preferred embodiment is particularly suitable for self-examination carried out in the convenience of the patient's home. In a preferred embodiment of the present invention a tonometer having an electro-optic unit and a control unit, is mounted on a frame that can be placed on a table. This frame has a curved support on which the patient leans his forehead for aligning the electro-optical unit with the eye. In such an embodiment, alignment of the equipment with the eye is confirmed by the medical staff, by watching the intensity level of detected reflected light.

Another variant consists of a plurality of electro-optical units, all linked to one central control unit consisting of a PC. The electro-optical units are mounted on a frame that allows several different patients to be examined and their IOP determined.

## Claims

1. A method of measuring internal pressure of a body having a convex surface comprising:
aligning with the body a longitudinal axis of a light collecting and delivering device (48) formed of a wall surrounding an axially extending lumen (50);
illuminating the body by way of the light collecting and delivering device and applying thereto a pneumatic pulse capable of substantially flattening the body, the illuminating light beam and the pneumatic pulse passing axially through the lumen (50),
guiding by total internal reflection through the wall of the light collecting and delivering device (48) light reflected from the body to a detector (72) when the body is in a non-flattened configuration, the light when the body is in a flattened configuration being reflected into the lumen (50) and prevented from reaching the detector (72); and
matching a time-related feature associated with changes in the light intensity of reflected light delivered to the detector, with a given pressure value related to the pneumatic pulse, the time related feature being at least one of a time interval in which the changes of the light intensity are detected and a slope of the changes of the light intensity.

2. The method as in claim 1, wherein the body is an eye (12, 42) and the aligning comprises centering a reticule image (76, 128) in a field of view of the eye.

3. A device for measuring internal pressure of a body, comprising:
a light collecting and delivering device (48) formed of a wall surrounding a lumen (50), the wall having an end surface for receiving light reflected by the body, the light being guided to travel along the wall by total internal reflection within the wall,
an illuminating beam source (126) for providing a light beam (60) to the body, the light beam passing through the lumen of the light collecting and delivering device;
an air source (54,124) for providing a pneumatic pulse of compressed air to the body by way of the lumen (50) of the light collecting and delivering device to flatten a convex surface of the body;
a light detector (72) mounted to receive light reflected by the body and passing through the wall of the light collecting and delivering device (48),
a reflector (58) provided between the lumen (50) and the light detector (72) to reflect the illuminating light beam (60) into the lumen (50) and to prevent light reflected by the body into the lumen (50) from reaching the detector (72), and
a control unit (30) connected to the detector (72) to provide a measure of the internal pressure of the body.

4. A device as claimed in claim 3, wherein the light collecting and delivering device (48) comprises unitary tubular body of transparent material.

5. A device as claimed in claim 4, wherein a light coupling device (56) is provided to couple the light passing through the light collecting and delivering device (48) to the detector (72).

6. A device as claimed in any of claims 3 to 5 wherein the end surface (52) of the light collecting and delivering body (48) for receiving light reflected from the body is provided with anti-reflection coating.

7. A device as claimed in any of claims 3 to 6, wherein the body is an eye (12, 42) and the device further comprises a reticule (76, 128) for projecting an image thereof on the eye (12, 42).

8. A device as claimed in any of claims 3 to 7, further comprising a mounting for securing to a head of a user.

9. A device as claimed in any of claims 3 to 8, wherein the control unit is operative to match a time-related feature associated with changes in the light intensity of reflected light delivered to the detector, with a given pressure value related to the pneumatic pulse, the time related feature being at least one of a time interval in which the changes of the light intensity are detected and a slope of the changes of the light intensity.

## Patentansprüche

1. Verfahren zum Messen eines Innendrucks eines Körpers mit einer konvexen Oberfläche, umfassend:
Ausrichten des Körpers an einer Längsachse einer Licht sammelnden und abgebenden Vorrichtung (48), die aus einer Wand besteht, die ein sich axial erstreckendes Lumen (50) umgibt;
Beleuchten des Körpers mittels der Licht sammelnden und abgebenden Vorrichtung und Beaufschlagen desselben mit einem pneumatischen Impuls, der in der Lage ist, den Körper im Wesentlichen abzuflachen, wobei der Beleuchtungslichtstrahl und der pneumatische Impuls axial durch das Lumen (50) hindurch verlaufen,
Lenken von Licht, das vom Körper reflektiert wird, durch Totalreflexion durch die Wand der Licht sammelnden und verteilenden Vorrichtung (48) zu einem Detektor (72), wenn der Körper in einer nicht-abgeflachten Gestalt vorliegt, wobei das Licht, wenn der Körper in einer abgeflachten Gestalt vorliegt, in das Lumen (50) zurückgeworfen wird und daran gehindert wird, den Detektor (72) zu erreichen; und
Abstimmen eines zeitabhängigen Merkmals, das mit Änderungen der Lichtstärke oder des reflektierten Lichts, das am Detektor ankommt, mit einem bestimmten Druckwert, der mit dem pneumatischen Druck in Beziehung steht, wobei das zeitabhängige Merkmal ein Zeitintervall, in dem die Änderungen der Lichtstärke erfasst werden, und/oder ein Gradient der Änderungen der Lichtstärke ist.

2. Verfahren nach Anspruch 1, wobei der Körper ein Auge (12, 42) ist und das Ausrichten das Zentrieren eines Fadenkreuzabbilds (76, 128) in einem Gesichtsfeld des Auges umfasst.

3. Vorrichtung zum Messen des Innendrucks eines Körpers, umfassend:
eine Licht sammelnde und abgebende Vorrichtung (48), die aus einer Wand besteht, die ein Lumen (50) umgibt, wobei die Wand eine Stirnfläche aufweist, um Licht aufzufangen, das von dem Körper reflektiert wird, wobei das Licht so gelenkt wird, dass es durch eine Totalreflexion innerhalb der Wand entlang der Wand wandert,
eine Beleuchtungsstrahlenquelle (126), die einen Lichtstrahl (60) zum Körper schickt, wobei der Lichtstrahl durch das Lumen der Licht sammelnden und abgebenden Vorrichtung verläuft;
eine Luftquelle (54, 124), die einen pneumatischen Impuls aus Druckluft mittels des Lumens (50) der Licht sammelnden und abgebenden Vorrichtung zum Körper schickt, um eine konvexe Oberfläche des Körpers abzuflachen;
einen Lichtdetektor (72), der so montiert ist, dass er Licht, das vom Körper reflektiert wird und durch die Wand der Licht sammelnden und abgebenden Vorrichtung (48) hindurch geht, auffängt,
einen Reflektor (58), der zwischen dem Lumen (50) und dem Lichtdetektor (72) vorgesehen ist, um den Beleuchtungslichtstrahl (60) in das Lumen (50) zu reflektieren und um zu verhindern, dass Licht, das vom Körper in das (Lumen) reflektiert wird, den Detektor (72) erreicht, und
eine Steuereinheit (30), die mit dem Detektor (72) verbunden ist, um ein Maß für den Innendruck des Körpers zu liefern.

4. Vorrichtung nach Anspruch 3, wobei die Licht sammelnde und abgebende Vorrichtung (48) einen einheitlichen Körper aus transparentem Material umfasst.

5. Vorrichtung nach Anspruch 4, wobei eine Lichtkopplungsvorrichtung (56) vorgesehen ist, um das Licht, das durch die Licht sammelnde und abgebende Vorrichtung (48) fällt, mit dem Detektor (72) zu verkuppeln.

6. Vorrichtung nach einem der Ansprüche 3 bis 5, wobei die Stirnfläche (52) des Licht sammelnden und abgebenden Körpers, die Licht auffängt, das vom Körper reflektiert wird, mit einer antireflektierenden Beschichtung versehen ist.

7. Vorrichtung nach einem der Ansprüche 3 bis 6, wobei der Körper ein Auge (12, 42) ist und die Vorrichtung ferner ein Fadenkreuz (76, 128) zum Projizieren eines Abbildes davon auf das Auge (12, 42) umfasst.

8. Vorrichtung nach einem der Ansprüche 3 bis 7, ferner eine Halterung zum Anbringen am Kopf eines Anwenders aufweisend.

9. Vorrichtung nach einem der Ansprüche 3 bis 8, wobei die Steuereinheit in der Lage ist, ein zeitabhängiges Merkmal, das mit Änderungen der Lichtstärke von reflektiertem Licht, das am Detektor ankommt, assoziiert ist, mit einem bestimmten Druckwert abzustimmen, der mit dem pneumatischen Impuls in Beziehung steht, wobei das zeitabhängige Merkmal ein Zeitintervall, in dem die Änderungen der Lichtstärke erfasst werden, und/oder ein Gradient der Änderungen der Lichtstärke ist.

## Revendications

1. Un procédé de mesure de la pression interne d'un corps possédant une surface convexe comprenant :
l'alignement avec le corps d'un axe longitudinal d'un dispositif de collecte et de diffusion de lumière (48) formé d'une paroi entourant un lumen s'étendant axialement (50),
l'éclairage du corps au moyen du dispositif de collecte et de diffusion de lumière et l'application à celui-ci d'une impulsion pneumatique capable d'aplatir sensiblement le corps, le faisceau lumineux d'éclairage et l'impulsion pneumatique passant axialement au travers du lumen (50),
le guidage, par une réflexion interne totale au travers la paroi du dispositif de collecte et de diffusion de lumière (48), de la lumière réfléchie à partir du corps vers un détecteur (72) lorsque le corps est dans une configuration non aplatie, la lumière, lorsque le corps est dans une configuration aplatie, étant réfléchie dans le lumen (50) et empêchée d'atteindre le détecteur (72), et
la mise en correspondance d'une caractéristique liée au temps associée à des variations dans l'intensité lumineuse de la lumière réfléchie fournie au détecteur, avec une valeur de pression donnée liée à l'impulsion pneumatique, la caractéristique liée au temps étant au moins une caractéristique parmi un intervalle temporel au cours duquel les variations de l'intensité lumineuse sont détectées et une pente des variations de l'intensité lumineuse.

2. Le procédé selon la Revendication 1, où le corps est un oeil (12, 42) et l'alignement comprend le centrage d'une image réticulaire (76, 128) d'un champ de vision de l'oeil.

3. Un dispositif de mesure de la pression interne d'un corps, comprenant :
un dispositif de collecte et de diffusion de lumière (48) formé d'une paroi entourant un lumen (50), la paroi possédant une surface d'extrémité destinée à recevoir de la lumière réfléchie par le corps, la lumière étant guidée de façon à se déplacer le long de la paroi par une réflexion interne totale à l'intérieur de la paroi,
une source de faisceau lumineux (126) destinée à fournir un faisceau lumineux (60) au corps, le faisceau lumineux passant au travers du lumen du dispositif de collecte et de diffusion de lumière,
une source d'air (54, 124) destinée à fournir une impulsion pneumatique d'air comprimé au corps au moyen du lumen (50) du dispositif de collecte et de diffusion de lumière de façon à aplatir une surface convexe du corps,
un détecteur lumineux (72) monté de façon à recevoir la lumière réfléchie par le corps et passant au travers de la paroi du dispositif de collecte et de diffusion de lumière (48),
un réflecteur (58) placé entre le lumen (50) et le détecteur de lumière (72) de façon à réfléchir le faisceau lumineux d'éclairage (80) dans le lumen (50) et à empêcher la lumière réfléchie par le corps dans le lumen (50) d'atteindre le détecteur (72), et
une unité de commande (30) raccordée au détecteur (72) de façon à fournir une mesure de la pression interne du corps.

4. Un dispositif selon la Revendication 3, où le dispositif de collecte et de diffusion de lumière (48) comprend un corps tubulaire monobloc d'un matériau transparent.

5. Un dispositif selon la Revendication 4, où un dispositif de couplage de lumière (56) est fourni de façon à coupler la lumière passant au travers du dispositif de collecte et de diffusion de lumière (48) au détecteur (72).

6. Un dispositif selon l'une quelconque des Revendications 3 à 5 où la surface d'extrémité (52) du dispositif de collecte et de diffusion de lumière (48) destinée à recevoir la lumière réfléchie à partir du corps est munie d'un revêtement antireflets.

7. Un dispositif selon l'une quelconque des Revendications 3 à 6, où le corps est un oeil (12, 42) et le dispositif comprend en outre un réticule (76, 128) destiné à projeter une image de celui-ci sur l'oeil (12, 42)

8. Un dispositif selon l'une quelconque des Revendications 3 à 7, comprenant en outre un support destiné à fixer une tête d'un utilisateur.

9. Un dispositif selon l'une quelconque des Revendications 3 à 8, où l'unité de commande est conçue de façon à mettre en correspondance une caractéristique liée au temps associée à des variations dans l'intensité lumineuse de la lumière réfléchie fournie au détecteur, avec une valeur de pression donnée liée à l'impulsion pneumatique, la caractéristique liée au temps étant au moins une caractéristique parmi un intervalle temporel au cours duquel les variations de l'intensité lumineuse sont détectées et une pente des variations de l'intensité lumineuse.
